# EUROPÄISCHE PATENTANMELDUNG

(11) **EP 1 468 968 A1**
(43) Veröffentlichungstag der Anmeldung: **20.10.2004**
(21) Anmeldenummer: 03450096.7
(22) Anmeldetag: 18.04.2003
(51) Int. Cl.: C02F 3/00, C12N 9/02, C12N 11/00

(54) **Eine Laccase enthaltender Biokatalysator**

(71) Anmelder: Technische Universitat, Institut fur Mikrobiologie und Abfalltechnologie, 8010 Graz (AT)
(72) Erfinder: Gübitz, Georg, Prof. Dr., 8047 Graz (AT); Cavaco-Paulo, Artur, Prof. Dr., 4715 1919 Braga (PT); Kandelbauer, Andreas, Dr., 8680 Mürzzuschlag (AT); Schroeder, Marc, Dipl.-Ing., 9753 Heinerscheid (LU); Held, Christof, Ingo, Dipl.-Ing., 8020 Graz (AT)
(74) Vertreter: Schwarz, Albin, Dr.

(57) **Zusammenfassung**

Zur chemischen Behandlung phenolischer Verbindungen und/oder aromatischer Amine, insbesondere Farbstoffe, wird ein Biokatalysator bereitgestellt, der dadurch gekennzeichnet ist, dass laccasehältige bakterielle Sporen in und/oder auf einem Träger immobilisiert sind Die bakteriellen Laccasen können im Gegensatz zu Pilzlaccasen auch bei höheren Temperaturen und pH-Werten eingesetzt werden.

## Beschreibung

Die Erfindung betrifft die Verwendung bakterieller Enzyme. Ferner betrifft die Erfindung einen Biokatalysator zur chemischen Behandlung phenolischer Verbindungen und/oder aromatischer Amine, insbesondere Farbstoffe, sowie ein Verfahren zum Entfärben von Textilprozesswässern und/oder -abwässern.

Unter chemischer Behandlung im Sinne der vorliegenden Erfindung werden zum Beispiel oxidative Abbaureaktionen und oxidative Kupplungen verstanden.

Phenolische Verbindungen und Aniline können unter dem Einfluss von Enzymen modifiziert und vollständig mineralisiert werden. Diese Klasse von Prozessen wird in diversen industriellen Anwendungen genutzt, z. B. zur Entfärbung von Farbstoffen und Pigmenten, in der Reinigung von Abwässern von Textilfabriken und Olivenverpressungsanlagen, bei der Behandlung von Wein oder der Biotransformation organischer Chemikalien.

Laccasen, oxidative Enzyme, die insbesondere von Weißfäulepilzen gebildet werden, sind besonders geeignet für großtechnische Anwendungen, da sie abgesehen von molekularem Sauerstoff als Cosubstrat keinerlei teure Cofaktoren wie NAD(P)H oder FADH benötigen und aufgrund ihrer breiten Substratspezifität vielfältig einsetzbar sind.

Es ist bereits seit einigen Jahren bekannt, dass Laccasen aus Pilzen zur Entfärbung von Prozesswässern eingesetzt werden können. In der US 5,795,855 A und von Call et al. (J. Biotechnol. 53 (1997), 163-202) wird der Einsatz von Laccasen, besonders unter Zusatz von niedermolekularen Mediatoren (GB 2 304 107 A), zur Entfärbung von ligninhaltigen Wässern, die in der Zellstoffindustrie oder bei der Papiererzeugung anfallen, beschrieben. Reaktoren zur Entfärbung von farbstoffhaltigen Abwässern aus der Textilindustrie wurden in der US 6,399,561 B beschrieben.

Die Fleckentfärbung von Textilien im Zuge der Reinigung von Kleidung kann ebenfalls durch den Zusatz von Laccasen erfolgen (GB 2 304 107 A). In ähnlicher Weise kann überschüssiger Farbstoff von nach verschiedenen Färbeverfahren gefärbten Textilien Laccase-katalysiert entfernt werden, wie in der WO 01/48304 A beschrieben. Die US 6,322,596 B beschreibt ein Verfahren, bei dem die Laccase-katalysierte Farbstoffentfärbung dazu ausgenutzt wird, gezielte Entfärbungsmuster auf vorgefärbten Stoffen zu erzeugen. Dabei wird über ein Inkjet-Printing-Verfahren eine Lösung mit einem Laccasepräparat auf das gefärbte Material an definierten Stellen aufgebracht, die sich entfärben und ein Muster auf dem gefärbten Untergrund hinterlassen.

Unter bestimmten Reaktionsbedingungen können Laccasen auch als Polymerisationskatalysatoren fungieren (Aktas et al., Bioresource Technology 80 (2001), 29-36; Ikeda et al., Macromolecules 29 (1996), 3053-3054) bzw. oxidative Kupplungsreaktionen herbeiführen (US 6,471,730 B). Laccasen können auch eingesetzt werden, um gefärbte Substanzen zu erzeugen. So ist etwa durch die oxidative Kupplung eines methylierten Hydroxyanthranilsäure-Derivats von Osiadacz et al. (J. Biotechnol. 72 (1999), 141-149) ein heterocyclischer Actinocinfarbstoff erzeugt worden.

Erst kürzlich wurde über die Verwendung von Laccasen zur Synthese einer Reihe von teilweise neuen Farbstoffen berichtet. Besonderes Interesse hat dabei die z.B. in der US 6,471,730 B, der EP 1 240 890 A, der WO 00/57848 A und der US 5,948,121 A beschriebene simultane in-situ Färbung von Keratinfasern durch ein Laccasepräparat auf sich gezogen. Eine analoge in-situ Färbeprozedur für Textilien wurde vor kurzem in der US 6,296,672 B vorgeschlagen.

Alle oben genannten Verfahren basieren auf der Verwendung von Laccasen aus Pilzen, die entweder mit Pilzen oder cloniert in Bakterien und Hefen (Jonsson et al., Curr. Genet. 32.6 (1997), 425-430; WO 01/92498 A) produziert werden.

Laccasen können als freie Enzyme eingesetzt werden, was allerdings den Nachteil hat, dass sie in Prozessen nicht im Kreislauf geführt und damit nicht mehrfach verwendet werden können. Deshalb basieren industrielle Prozesse auf der Verwendung von immobilisierten Laccasen.

Laccasen, die aus unterschiedlichen Weißfäulepilzen gewonnen werden können, sind in Umgebungen von saurem bis neutralem pH bei moderaten Temperaturen, d.h. unterhalb von 50°C, einsetzbar. Für eine Vielzahl von Anwendungen wäre es jedoch von Vorteil, auch über Laccasesysteme zu verfügen, die im Alkalischen und bei erhöhter Temperatur noch hohe Aktivitäten zeigen. Pilzlaccasen werden unter solchen Bedingungen in der Regel inaktiviert und zerstört.

Gewöhnlich kommen für die Biotransformationen und enzymatischen Abbaureaktionen lebende Pilze zum Einsatz. Größter Nachteil dieser Methode ist neben der Beschränkung auf physiologische Umgebungstemperaturen und pH-Werte beispielsweise des Abwassers die zum Zellwachstum benötigte kontinuierliche Zuführung von Nährstoffen und Mineralien. Zudem werden neben der gewünschten Laccase immer auch andere, in der Regel unerwünschte Stoffwechselprodukte in das umgebende Medium ausgeschieden.

Die Erfindung bezweckt die Überwindung der oben genannten Nachteile und Schwierigkeiten und stellt sich die Aufgabe, Laccasen zur chemischen Behandlung phenolischer Verbindungen und/oder aromatischer Amine bereitzustellen sowie einen Biokatalysator, der bei höheren Temperaturen und pH-Werten eingesetzt werden kann und weder Nährstoffe benötigt, noch unerwünschte Nebenprodukte erzeugt. Ferner soll ein Verfahren zum Entfärben von Textilprozesswässern und/oder - abwässern vorgesehen werden.

Diese Aufgabe wird erfindungsgemäß durch die Verwendung bakterieller Laccasen, insbesondere in Form laccasehältiger bakterieller Sporen gelöst.

Vorzugsweise werden die bakteriellen Laccasen zum Entfärben eingesetzt, wobei die Farbstoffe oxidativ abgebaut werden.

In bevorzugter Weise werden die bakteriellen Laccasen zur Synthese von Farbstoffen verwendet, wobei die Synthese über eine oxidative Kupplungsreaktion erfolgt.

Von Diamantidis et al. (Soil Biology and Biochemistry 32.7 (2000), 919-927) sowie Martins et al. (J. Biol. Chem. 277.21 (2002), 18849-18859) wurden Laccasen auch schon als originär in Bakterien nachgewiesen und sowohl hinsichtlich ihrer physikalisch-chemischen Eigenschaften als auch ihrer genetischen Provenienz charakterisiert. Die Anwendung bakterieller Laccasen oder laccasehältiger bakterieller Sporen zur chemischen Behandlung, insbesondere zum oxidativen Abbau oder zur oxidativen Kupplung phenolischer Verbindungen und/oder aromatischer Amine, besonders von Farbstoffen, wurde jedoch nicht beschrieben.

Im Gegensatz zu Laccasen von Weißfäulepilzen weisen bakterielle Laccasen zumeist pH-Optima im alkalischen Bereich auf und sind auch unempfindlicher gegenüber extremeren Temperaturen.

Der erfindungsgemäße enzymatische Biokatalysator ist dadurch gekennzeichnet, dass laccasehältige bakterielle Sporen in und/oder auf einem Träger immobilisiert sind.

Der erfindungsgemäße Biokatalysator macht sich originär von Bakterien gebildete und an der Sporenoberfläche immobilisierte Phenoloxidasen zunutze und ermöglicht damit die Anwendung einer einfach handhabbaren Biokatalysatorform mit neuem Eigenschaftsprofil hinsichtlich der Stabilität und der anwendbaren Reaktionsbedingungen im industriellen Umfeld von Textilindustrie, Papier- und Zellstoffindustrie sowie ähnlichen Industrien, bei deren Tätigkeiten Verbindungen entstehen, die potentiell durch Einwirkung von Laccasen oxidierbar sind.

Vorzugsweise ist der Träger für die laccasehältigen Sporen ausgewählt aus der Gruppe bestehend aus natürlichen und synthetischen Schwämmen, pulverisierten Cellulosematerialien, Holzrinde, Calciumalginat-Gelen, Aluminiumoxid, Glas, natürlichen und synthetischen Polymeren und Copolymeren.

Die Immobilisierung ist bevorzugt durch mindestens eines, ausgewählt aus der Gruppe bestehend aus Absorption, Einschluss und kovalente Bindung erfolgt, wobei die kovalente Bindung vorzugsweise über funktionelle Gruppen, ausgewählt aus der Gruppe bestehend aus Aminen, Alkoholen, Hydroxiden, Thiolen, Carbonylen und Epoxiden, erfolgt ist.

In einer bevorzugten Ausführungsform sind die Sporen mittels eines Polymerspacers, ausgewählt aus der Gruppe bestehend aus Polyethylenglykol, Polyethoxyethylen, BSA, Cellulose, Dextran und Xylan, kovalent an den Träger gebunden.

Vorzugsweise stammen die Sporen von laccasetragenden *Bacillus sp.* oder *Clostridien sp.,* insbesondere *Bacillus subtilis.*

Das erfindungsgemäße Verfahren zum Entfärben von Textilprozesswässern und/oder abwässern ist dadurch gekennzeichnet, dass die Prozess- und/oder Abwässer mit einem erfindungsgemäßen Biokatalysator in Kontakt gebracht werden.

Die harten Bedingungen, die üblicherweise in Textilprozesswässern vorherrschen, wirken sich aufgrund der Eigenschaften der bakteriellen Laccasen weitaus weniger drastisch aus, wohingegen Pilzsysteme für solche Anwendungen nahezu gänzlich ungeeignet sind. Im Zusammenhang mit der Anwendung von Sporen wirken sich die äußerst lebensfeindlichen Bedingungen sogar positiv aus, da sie das Auskeimen der Sporen verhindern. Die Immobilisierung der Sporen auf geeigneten Trägermaterialien liefert folglich eine einfache und praktikable Lösung für das Problem kontinuierlicher Biotransformationen, insbesondere in der Aromatenmodifikation.

Gemäß einer bevorzugten Ausführungsform werden die Prozess- und/oder Abwässer in einem Rohrreaktor mit dem Biokatalysator in Kontakt gebracht und kontinuierlich entfärbt.

Besonders vorteilhaft ist das Verfahren, wenn das entfärbte Abwasser im Färbeprozess wiederverwendet wird.

Eine Kombination des erfindungsgemäßen Biokatalysators mit Mediatoren (redoxaktiven Substanzen) ist ebenfalls öglich. Der Anwendungsbereich der laccasehältigen bakteriellen Sporen kann dadurch noch erweitert werden.

Die Erfindung wird nachfolgend anhand von Beispielen näher erläutert.

### Beispiele:

### Gewinnung der laccasehältigen Sporen

Im Zuge eines groß angelegten Screeningverfahrens nach Laccase produzierenden Bakterien wurde eine Reihe von Stämmen identifiziert, in deren Sporen Laccasen nachweisbar waren. Die Bakterien wurden demgemäss bevorzugt auf einem Sporulationsmedium gezüchtet. Für die Anwendung bei hohen pH-Werten und Temperaturen erwiesen sich insbesondere *Bacillus sp.* mit einem pH-Optimum von 10 und einem Temperaturoptimum von 65°C als geeignet für die Produktion laccasehältiger Sporen.

Nach Inkubationszeiten zwischen 24 und 36 Stunden wurden nur Sporen im Fermentationsmedium nachgewiesen. Die bakteriellen Sporen wurden geerntet und vom Kultivationsmedium durch Zentrifugation bei 11.000 g bzw. über Filtration mit 0,45 µm - Filtern abgetrennt. Die Laccaseaktivität der Sporen erwies sich sowohl gegenüber alkalischen pH-Werten als auch gegenüber erhöhter Temperatur als stabil. Die Halbwertszeit der Laccaseaktivität der *Bacillus subtilis* - Sporen betrug so beispielsweise bei pH 9,3 und 80°C 25 Minuten. Die Laccaseaktivität erscheint gegenüber hohen pH-Werten weitgehend unempfindlich. Anschließend wurden die Sporen gewaschen und wie nachfolgend beschrieben immobilisiert. Der angeführte Immobilisierungsprozess verhindert die Keimung der Sporen unter den Bedingungen des oxidativen Abbaus bzw. der Biotransformation.

### Immobilisierung der Sporen

Im Fall des vorzugsweise eingesetzten γ-Aluminiumoxids wurden Aminogruppen mit Hilfe von Triethoxysilanpropylamin in Aceton auf der Oberfläche des Trägers verankert. Als Vernetzungsreagens diente ein bifunktionales Aldehyd, wie etwa Glutaraldehyd oder höhere Homologe. Auch andere bifunktionelle reaktive Reagenzien können verwendet werden, wie zum Beispiel Aldehyddextran, Aminodextran, Polyethylenglykoldiamin, etc.

Die Sporen wurden gereinigt und in den folgenden Verfahrensschritten als Pulver eingesetzt. Die Sporen wurden mittels Glutaraldehyd oder höheren Homologen. vorzugsweise bei einem pH zwischen 7 und 9, kovalent an den Träger gebunden.

### Beispiel 1

Aluminiumoxid-Kugeln wurden zweimal in einer 6% (v/v) Aminopropyltriethoxysilan-Aceton-Lösung für 18 Stunden auf 60°C erhitzt. Die silanisierten Kugeln wurden zweimal mit destilliertem Wasser gewaschen und für zwei Stunden bei 20°C in eine 10% (v/v) wässrige Glutaraldehydlösung gegeben.

Ferner können Sporen mittels eines Polymerspacer kovalent an einen Träger gebunden werden. Als Spacer können synthetische Polymere, wie Polyethylenglykol, Polyethoxyethylen, etc., und natürliche Materialien, wie BSA, und diverse Polysaccharide (Cellulose, Dextran, Xylan, etc.) genutzt werden.

Die funktionellen Endgruppen des Polymers müssen aktiviert werden. Hierzu können unterschiedliche Reagenzien, wie z.B. Epichlorhydrin, Carbonyldiimidazol oder Cyanurchlorid, verwendet werden. Vorteilhaft dabei sind Hydroxygruppen, es können aber auch freie Amingruppen genutzt werden.

### Beispiel 2

4 g PEG (Polyethylenglykol) und 2 g 1,1'-Carbonyldiimidazol wurden in 100 ml trockenem Dimethylsulfoxid suspendiert und für 5 Stunden bei Raumtemperatur gerührt. Anschließend wurde das aktivierte Polymer filtriert, gewaschen und unter Vakuum getrocknet.

Die Länge des Spacers kann mittels des Molekulargewichts des Polymers definiert werden und ist abhängig von der Art der zu immobilisierenden Sporen.

Das aktivierte Polymer wurde in schwach basischem Medium an den Träger angehängt.

### Beispiel 3

Unterschiedliche Konzentrationen (zwischen 0,01 und 0,1 g/ml) des aktivierten Polymers wurden eingesetzt, um eine homogene und optimale Beladung der Trägeroberfläche zu erzielen. Die Sporen wurden mit vorgelegtem modifiziertem Träger in einem entsprechenden Puffer suspendiert. Die Reaktionslösung wurde über Nacht bei 4°C leicht geschüttelt, anschließend filtriert und mit Kaliumphosphat-Puffer gewaschen. Die immobilisierten Sporen wurden unter Kühlung aufbewahrt.

### Entfärbung von Prozesswasser

### Beispiel 4

Textil-Färbeabwässer, welche 50 mg/l Diamond-Black als Hauptfarbstoff enthielten, wurden in einem Rohrreaktor mit immobilisierten Sporen behandelt. Ein Liter dieses Abwassers wurde bei pH 9 und einer Temperatur von 60°C innerhalb von 35 Minuten entfärbt. Dieses entfärbte Abwasser wurde zum Färben mit Reaktivfarbstoffen - CI Reactive Orange 70 und CI Reactive Blue 214 - anstelle von Frischwasser eingesetzt. Die Farbstoffe wurden auf gebleichten Baumwollstoffen in zwei Abstufungen eingesetzt: schwach - 0,5%, bezogen auf das Stoffgewicht und stark - 3%, bezogen auf das Stoffgewicht, mit 20 g/l Na₂CO₃ und 60 g/l Na₂SO₄, Flüssigkeitsverhältnis 1:20.

Der Farbunterschied, bezogen auf Stoffe, die mit Frischwasser gefärbt wurden, ergab bei 0,5% ein ΔE* von 0,912 für Reactive Orange 70 und ein ΔE* von 0,174 für Reactive Blue 214. Für eine Farbabstufung von 3% war ΔE* 0,342 für Reactive Orange 70 und 0,129 für Reactive Blue 214.

### Beispiel 5

Textil-Färbeabwässer, welche 50 mg/l Diamond Fast Brown als Hauptfarbstoff enthielten, wurden in einem Rohrreaktor mit immobilisierten Sporen behandelt. Ein Liter dieses Abwassers wurde bei pH 9 und einer Temperatur von 60°C innerhalb von 50 Minuten entfärbt. Dieses entfärbte Abwasser wurde zum Färben mit Reaktivfarbstoffen - CI Reactive Orange 70 und CI Reactive Blue 214 - anstelle von Frischwasser eingesetzt. Die Farbstoffe wurden auf gebleichten Baumwollstoffen in zwei Abstufungen eingesetzt: schwach - 0,5%, bezogen auf das Stoffgewicht und stark - 3%, bezogen auf das Stoffgewicht, mit 20 g/l Na₂CO₃ und 60 g/l Na₂SO₄, Flüssigkeitsverhältnis 1:20.

Der Farbunterschied, bezogen auf Stoffe, die mit Frischwasser gefärbt wurden, ergab bei 0,5% ein ΔE* von 1,105 für Reactive Orange 70 und ein ΔE* von 0,640 für Reactive Blue 214. Für eine Farbabstufung von 3% war ΔE* 0,731 für Reactive Orange 70 und 0,319 für Reactive Blue 214.

### Beispiel 6

Textil-Färbeabwässer, welche 50 mg/l Indigo als Hauptfarbstoff enthielten, wurden mit immobilisierten Sporen behandelt. Ein Liter dieses Abwassers wurde bei pH 9 und einer Temperatur von 60°C innerhalb von 65 Minuten entfärbt. Dieses entfärbte Abwasser wurde zum Färben mit Reaktivfarbstoffen - CI Reactive Orange 70 und CI Reactive Blue 214 - anstelle von Frischwasser eingesetzt. Die Farbstoffe wurden auf gebleichten Baumwollstoffen in zwei Abstufungen eingesetzt: schwach - 0,5%, bezogen auf das Stoffgewicht und stark - 3%, bezogen auf das Stoffgewicht, mit 20 g/l Na₂CO₃ und 60 g/l Na₂SO₄, Flüssigkeitsverhältnis 1:20.

Der Farbunterschied, bezogen auf Stoffe, die mit Frischwasser gefärbt wurden, ergab bei 0,5% ein ΔE* von 2,726 für Reactive Orange 70 und ein ΔE* von 1,465 für Reactive Blue 214. Für eine Farbabstufung von 3% war ΔE* 0,853 für Reactive Orange 70 und 0,699 für Reactive Blue 214.

Die Ergebnisse zeigen, dass mit Hilfe des erfindungsgemäßen Verfahrens entfärbte Textilprozesswässer und/oder -abwässer vorteilhaft rückgeführt und erneut im Färbeprozess eingesetzt werden können.

### Synthese von Farbstoffen unter oxidativer Kupplung

### Beispiel 7

Lösungen mit 1 g/l Indigo Carmine wurden in Gegenwart von 100 mg/l Isatin-5-sulfonsäure bei 45°C und einem pH-Wert > 5 für drei Stunden mit immobilisierten Sporen behandelt. Dabei wurde aus dem blaugefärbten Indigo Carmine ein neuer roter Farbstoff erzeugt. Das entsprechende oxidative Kopplungsprodukt mit einem um 249 Einheiten vergrößerten Molekulargewicht wurde mittels einer Kombination von Flüssigkeitschromatographie und Massenspektroskopie nachgewiesen.

### Beispiel 8

Die zwei wasserlöslichen Farbstoffzwischenstufen, 4-Aminodiphenylamin-2-sulfonsäure (CAS 91-30-5) und 5-Amino-2-naphthalensulfonsäure (CAS 199-79-9), wurden in einem kontinuierlichen Verfahren mit immobilisierten Sporen bei 80°C, einem pH-Wert > 5 und einer Verweilzeit von 45 Minuten behandelt. Dabei wurden ein brauner und ein blauer zur Färbung von Textilien geeigneter Farbstoff erhalten. Durch oxidative Kupplung der Farbstoffvorstufen wurden auch rote, violette und dunkelblaue Produkte erhalten, welche ebenfalls zur Färbung von Stoffen geeignet sind.

## Patentansprüche

1. Verwendung bakterieller Laccasen zur chemischen Behandlung phenolischer Verbindungen und/oder aromatischer Amine, insbesondere Farbstoffe.

2. Verwendung nach Anspruch 1 zur Entfärbung, wobei die chemische Behandlung eine oxidative Abbaureaktion ist.

3. Verwendung nach Anspruch 1 zur Synthese von Farbstoffen, wobei die chemische Behandlung eine oxidative Kupplung ist.

4. Verwendung nach einem der Ansprüche 1 bis 3 in Form laccasehältiger bakterieller Sporen.

5. Biokatalysator zur chemischen Behandlung phenolischer Verbindungen und/oder aromatischer Amine, insbesondere Farbstoffe, **gekennzeichnet durch** in und/oder auf einem Träger immobilisierte laccasehältige bakterielle Sporen.

6. Biokatalysator nach Anspruch 5, **dadurch gekennzeichnet, dass** der Träger ausgewählt ist aus der Gruppe bestehend aus natürlichen und synthetischen Schwämmen, pulverisierten Cellulosematerialien, Holzrinde, Calciumalginat-Gelen, Aluminiumoxid, Glas, natürlichen und synthetischen Polymeren und Copolymeren.

7. Biokatalysator nach Anspruch 5 oder 6, **dadurch gekennzeichnet, dass** die Immobilisierung durch mindestens eines, ausgewählt aus der Gruppe bestehend aus Absorption, Einschluss und kovalente Bindung, erfolgt ist.

8. Biokatalysator nach Anspruch 7, **dadurch gekennzeichnet, dass** die kovalente Bindung über funktionelle Gruppen, ausgewählt aus der Gruppe bestehend aus Aminen, Alkoholen, Hydroxiden, Thiolen, Carbonylen und Epoxiden, erfolgt ist.

9. Biokatalysator nach Anspruch 7 oder 8, **dadurch gekennzeichnet, dass** die Sporen mittels eines Polymerspacers, ausgewählt aus der Gruppe bestehend aus Polyethylenglykol, Polyethoxyethylen, BSA, Cellulose, Dextran und Xylan, kovalent an den Träger gebunden sind.

10. Biokatalysator nach einem der Ansprüche 5 bis 9, **dadurch gekennzeichnet, dass** die Sporen von laccasetragenden *Bacillus sp.* oder *Clostridien sp.,* insbesondere *Bacillus subtilis,* stammen.

11. Verfahren zum Entfärben von Textilprozesswässern und/oder -abwässern, **dadurch gekennzeichnet, dass** die Prozess- und/oder Abwässer mit einem Biokatalysator gemäß einem der Ansprüche 5 bis 10 in Kontakt gebracht werden.

12. Verfahren gemäß Anspruch 11, **dadurch gekennzeichnet, dass** die Prozess- und/oder Abwässer in einem Rohrreaktor mit dem Biokatalysator in Kontakt gebracht und kontinuierlich entfärbt werden.

13. Verfahren nach Anspruch 11 oder 12, **dadurch gekennzeichnet, dass** das entfärbte Abwasser im Färbeprozess wiederverwendet wird.
